# EUROPÄISCHE PATENTSCHRIFT

(11) **EP 2 486 945 B1**
(45) Veröffentlichungstag und Bekanntmachung des Hinweises auf die Patenterteilung: **22.01.2014**
(21) Anmeldenummer: 11001127.7
(22) Anmeldetag: 11.02.2011
(51) Int. Cl.: A61L 2/18, A61B 19/00, A61C 19/00

(54) **Verfahren und Vorrichtung zur Reinigung von medizinischen Instrumenten**
Method and device for cleaning medical instruments
Procédé et dispositif de nettoyage d'instruments médicaux

(43) Veröffentlichungstag der Anmeldung: 15.08.2012
(73) Patentinhaber: Oro Clean Chemie AG, 8320 Fehraltorf (CH)
(72) Erfinder: Ionidis, Georgios, 8052 Zürich (CH)

(56) Entgegenhaltungen:
- EP-A1- 1 779 800
- DE-A1- 19 913 943
- US-A- 5 165 503
- US-A- 5 723 090
- US-A- 5 755 894
- US-A- 5 795 404

## Beschreibung

Die vorliegende Erfindung betrifft eine Vorrichtung und ein Verfahren zur Reinigung und/oder Desinfektion und/oder der Pflege von medizinischen, insbesondere zahnärztlichen Instrumenten mit einem Hohlraum, insbesondere zahnärztlichen Präparationsinstrumenten mit Antriebselemente für rotierende Instrumente, wie beispielsweise zahnärztliche Handstücken, Winkelstücken und/oder Turbinen. In der Zahnmedizin kommt es zu einem intensiven Gebrauch von Präparationsinstrumenten, mit welchen unter Verwendung von rotierenden Bohr-, Schleif- und Polierkörpern Zähne behandelt werden. Bei diesem Gebrauch dringen Blut, Speichel, Wasser, Schleif- und/oder Polierkörper in das Innere des medizinischen Instruments ein und verunreinigen dieses. Diese Verunreinigungen können beim weiteren Gebrauch mit dem Patienten in Berührung kommen und können beim Patienten zu Infektionen führen. Diese Infektionen gilt es zu vermeiden. Die angesaugten Verunreinigungen führen zusätzlich zur Ablagerungen und Biofilmwachstum, die durch Abrasion oder Veklemmungen Präzisionsteile schädigen und die Lebensdauer der medizinischen Instrumente verkürzen. Ein weiteres Problem bei Gerbrauch der Instrumente, liegt darin begründet, dass diese Instrumente wasserführende Systeme aufweisen. In diesen wasserführenden Systemen kann es zu Kalkablagerungen kommen, die das wasserführende System verstopft. Aus diesen Gründen muss das Innere des medizinischen Instruments in regelmässigen Abständen gereinigt und desinfiziert werden.

Aus US 5 755 894 ist ein Zubehörteil zu einer Spritze bekannt. Mittels dieses Zubehörteils ist es möglich Flüssigkeiten oder Gase aus der Spritze durch das zu reinigende Instrument zu spülen und dadurch zu reinigen. Die in der Spritze enthaltende Gasmenge ist jedoch zu klein um das Innere des zu reinigenden Instruments zu trocknen.

Aus DE 43 26 956 ist eine Verfahren zur Reinigung von zahnärztlichen Instrumenten mit einem Hohlraum bekannt. Bei diesem Verfahren wird das zu reinigende Instrument in einem mit mindestens zwei Öffnungen versehenen Gehäuse angeordnet. Dabei ist das Gehäuse über eine Öffnung am Vakuumnetz der zahnärztlichen Praxis angeschlossen. Durch eine zweite Öffnung wird Umgebungsluft angesaugt. Im Gehäuse befindet sich zusätzlich eine Reinigungsflüssigkeit. Das zu reinigende Instrument ist derart im Gehäuse angeordnet, dass beim Reinigungsvorgang die Umgebungsluft im Gehäuse Reinigungsflüssigkeit mitreist und diese durch den Hohlraum des zahnärztlichen Instruments in das Vakuumnetz strömt. Auf diese Weise wird das Instrument gereinigt. Der Reinigungseffekt kann zusätzlich durch eine Rotation des Instruments verstärkt werden. Dieses Verfahren birgt den Nachteil, dass beim Reinigen immer ein Gemisch aus Luft und Reinigungsmittel in den Hohlraum gebracht wird. Es ist nicht kontrollierbar ob genügend Reinigungsmittel in den Hohlraum gebracht wurde. Ein weiterer Nachteil besteht darin, dass das Reinigungs- und Desinfektionsmittel unkontrolliert durch die zweite Öffnung austreten kann und so die zahnärztliche Praxis verunreinigt werden kann. Ein weiterer Nachteil besteht darin, dass Reinigungsmittel die vorhandene Schmierflüssigkeit entfernt. Aus diesem Grund muss nach dem Reinigen das Instrument geölt werden. Bei dem in DE 43 26 956 vorgestellten Verfahren wird dazu in einem zweiten Schritt der Behälter gereinigt und mit Öl gefüllt. Anschliessend wird der Behälter erneut an das Vakuumnetz angeschlossen und ein Luft-Öl-Gemisch in das Instrument gebracht. Dieser Vorgang ist aufwändig. Ausserdem ist die Anschaffung und die Wartung solchen Geräte sehr teuer.

Aufgabe der Erfindung ist es deshalb eine Reinigungsvorrichtung und Verfahren zur Reinigung von medizinischen Instrumenten zur Verfügung zu stellen, das eine zuverlässige Reinigung, Desinfektion und Pflege des medizinischen Instruments sicherstellt und das einfach anzuwenden und günstig in der Herstellung und der Anwendung ist.

Diese Aufgabe wird durch eine Reinigungsvorrichtung und einem Verfahren zur Reinigung eines medizinischen Instruments gelöst, welche die im unabhängigen Patentanspruch angegebenen Merkmale aufweist. Weitere vorteilhafte Ausführungsformen können den abhängigen Ansprüchen entnommen werden.

Die erfindungsgemässe Reinigungsvorrichtung ist zur Reinigung von medizinischen Instrumenten mit mindestens einem Instrumentenhohlraum und mindestens zwei Öffnungen bestimmt. Dabei weist die Reinigungsvorrichtung ein erstes Befestigungsmodul zur lösbaren Befestigung des medizinischen Instruments und ein zweites Befestigungsmodul zur lösbaren Befestigung eines ersten Behälters zur Aufnahme von Flüssigkeit und/oder Luft auf. Die Reinigungsvorrichtung weist einen Reinigungsvorrichtungshohlraum auf, der derart ausgestaltet ist, dass bei befestigtem medizinischem Instrument und befestigtem Behälter die Flüssigkeit und/oder die Luft vom Innern des Behälters durch den Reinigungsvorrichtungshohlraum in den Instrumentenhohlraum spülbar ist. Die Reinigungsvorrichtung weist eine erste und eine zweite Betriebsposition auf, wobei in der ersten Betriebsposition die Flüssigkeit aus dem Behälter durch den Reinigungsvorrichtungshohlraum in den Instrumentenhohlraum spülbar ist und in der zweiten Betriebsposition Luft aus der Umgebung durch den Behälter und den Reinigungsvorrichtungshohlraum in den Instrumentenhohlraum spülbar ist.

Die erfindungsgemässe Reinigungsvorrichtung ist insbesondere für zahnärztliche Instrumente, wie beispielsweise zahnärztliche Handstücken, Winkelstücken oder Turbinen geeignet. Die Reinigungsvorrichtung kann jedoch auch für die Reinigung von anderen medizinischen oder veterinärmedizinischen Instrumenten, wie beispielsweise Endoskopen, Kanülen oder andere Instrumenten mit Hohlräumen, verwendet werden.

In der Regel weist der Instrumentenhohlraum genau zwei Öffnungen auf. Beim Reinigungsvorgang wird durch eine Öffnung die Reinigungsflüssigkeit in den Instrumentenhohlraum gespült und die Reinigungsflüssigkeit tritt aus der zweiten Öffnung aus dem Instrumentenhohlraum aus. Auf diese Weise wird der Instrumentenhohlraum mit der Reinigungsflüssigkeit durchspült.

Meistens weist das medizinische Instrument genau einen Instrumentenhohlraum auf, der gereinigt werden muss. Manche Instrumente weisen jedoch mehr als einen Instrumentenhohlraum auf, Es gibt beispielweise Instrumente mit zusätzlichen Luft- und / oder Wasserkanälen die parallel zum ersten Instrumentenhohlraum verlaufen.. Instrumentenhohlräume können auch in Form eines Kanals ausgestaltet sein. Es ist auch möglich, dass das Instrument einen Kanal zum Einbringen einer Sonde aufweist. Manche der Kanäle enden bei Düsen, die eine im Kanal fliessende Flüssigkeit an einen bestimmten Ort transportiert. Beispielweise sind oft zahnärztliche Handstücke, Winkelstücke oder Turbinen so konstruiert, dass Wasser aus Innenkanälen durch eine Düse an einen Zahn gespritzt wird, um diesen während des Bohrens zu kühlen. Diese zusätzlichen Instrumentenhohlräume, auch Kanäle genannt, sind ebenfalls von Verunreinigung betroffen und können ebenfalls mit der erfindungsgemässen Reinigungsvorrichtung gereinigt werden. Dazu müssen die zusätzlichen Instrumentenhohlräume ebenfalls am ersten Befestigungsmodul mit dem Reinigungsvorrichtungshohlraum verbindbar sein. Beim Reinigungsvorgang werden die Instrumentenhohlräume gleichzeitig mit der Flüssigkeit, die sich zu zuvor im befestigten Behälter befunden hat, gespült.

Bevorzugterweise ist die Reinigungsvorrichtung so konstruiert, dass im Inneren keine Lücken und/oder Toträume bestehen, in welchen während des Spülens mit Luft in der zweite Betriebsposition Flüssigkeit in diesen Lücken oder Toträumen verbleiben können. Eine solche Konstruktion verhindert dass beim Spülen mit Luft kleine Mengen an Flüssigkeit aus diesen Lücken und/oder Toträumen in das medizinische Instrument eintreten und die Dauer der Entleerung verlängert wird.

Bevorzugterweise ist die Reinigungsvorrichtung so konstruiert, dass es möglich ist Luft zur Unterdruckausgleich bei Dispersion von Flüssigkeit oder Luft aus der Umgebung in den Behälter in der ersten als auch in der zweiten Betriebsposition anzusaugen, dabei ist in der erste Position der Abfluss von Flüssigkeit aus dem Behälter verhindert. Dies kann dadurch erreicht werden, indem die Luftzuführkanäle schmal genug sind um den Fluss von Flüssigkeit auf Grund von Oberflächenspannung und/oder Affinität des Materials der Luftzufuhrkanäle zu verhindern. Von Vorteil ist ein Luftzufuhrkanal mit mindestens einem Winkel bzw. Biegung, weil eine Winkel bzw. Biegung den Abfluss zusätzlich erschwert. Durch geeignete Anpassung der Länge der Luftzufuhrkanäle und der Anzahl der Winkel ist es möglich ein Austreten der Flüssigkeit zu verhindern, wobei Luft immer noch durchströmen kann. Die Öffnung der Luftzufuhrkanäle gegen die Atmosphäre kann durch einen Sterilfilter abgedeckt werden. Der Sterilfilter sollte eine Porenweite von kleiner als 0.2 mm aufweisen um das Eindringen von Bakterien zu unterbinden.

Als Behälter können unterschiedliche Aufnahmevorrichtungen für Flüssigkeiten verwendet werden. Es können beispielsweise starre Behälter aus Metall, Glas oder Kunststoff verwendet werden. Prinzipiell sollen nur starre oder nur leicht verformbare Behälter verwendet sein, die während die Anwendung nicht zusammenfalten um das Notwendige Unterdruck für Lufteinlas zu kreieren. Es sind jedoch auch weitere Ausgestaltungen für den Behälter möglich. Es können Beispielweise Behälter mit einer zusätzlichen, wiederverschliessbaren Öffnung verwendet werden durch welche die Flüssigkeit nachgefüllt werden kann. Es ist auch grundsätzlich möglich diesen Behälter mit einem Reservoir mit Hilfe eines Schlauches permanent oder zeitweise zu verbinden um die Lösung nachfüllen.

Idealweise, ist der Behälter mit Hilfe eines reversible entfernbaren Gewindeeinsatzes an das Befestigungsmodul fixiert, in das einfach ein anderes Teil mit beispielsweise einem anderen Gewindedurchmesser eingesetzt werden kann. Dies bietet den Vorteil, dass auf die Reinigungsvorrichtung verschiedene Behälter verwendet werden können, bzw. auf einfache und schnelle Weise umgerüstet werden kann.

Es ist möglich, dass ein zweiter Behälter am zweiten Befestigungsmodul befestigt werden kann und das zweite Aufnahmeelement ein Auswahlmodul aufweist, das die wahlweise Entnahme von Flüssigkeit aus dem ersten oder zweiten Behälter ermöglicht. So kann ein Behälter eine Reinigungsflüssigkeit und ein zweiter Behälter Desinfektionsflüssigkeit erhalten. Durch Bewegung der drehbaren Scheibe kann gewählt werden welche der Flüssigkeiten durch die Dosierpumpe gepumpt werden soll. Der Schritt des Ausblasens mit Luft bleibt bei dieser Ausführungsform gleich.

Idealerweise wird eine Flüssigkeit verwendet, deren Dichte grösser als die Dichte von atmosphärischer Luft ist. Die Betriebspositionen des Reinigungsinstruments sind so ausgewählt, dass in der ersten Betriebsposition sich die Flüssigkeit vor dem zweiten Befestigungsmodul befindet und in der zweiten Betriebsposition sich die atmosphärische Luft vor dem zweiten Befestigungsmodul befindet. Bevorzugterweise weist die Vorrichtung eine Dosierpumpe auf, wobei mittels der Dosierpumpe in der ersten Betriebsposition Flüssigkeit aus dem Behälter durch den Reinigungsvorrichtungshohlraum in den Instrumentenhohlraum spülbar ist und mittels der Dosierpumpe in der zweiten Betriebsposition Luft aus der Umgebung durch den Behälter und den Reinigungsvorrichtungshohlraum in den Instrumentenhohlraum spülbar ist. Die Dosierpumpe kann ein festes Volumen haben, welche eine stets gleiche Menge an Flüssigkeit abgibt. Alternativ, ist es vorstellbar, eine Dosierpumpe mit verstellbares Volumen (z.B. Durch Begrenzung des Kolbenwegs), deren Abgabevolumen variiert werden kann zu verwenden.

Idealerweise wird durch eine Verschwenkung der Reinigungsvorrichtung um näherungsweise 180° in Schwerkraftrichtung zwischen der ersten und zweiten Betriebsposition gewechselt. Dabei ist in der ersten Betriebsposition die Flüssigkeit auf Grund der Schwerkraft durch das Aufnahmemodul spülbar. Die Flüssigkeit wird unter Einfluss der Dosierpumpe gezogen und fliesst dabei vom Behälter durch die Reinigungsvorrichtung durch den Instrumentenhohlraum und tritt aus einer Öffnung des Instrumentenhohlraums aus. Auf diese Weise werden sämtliche Hohlräume mit Flüssigkeit gefüllt. Nachdem genügend Flüssigkeit durch den Instrumentenhohlraum gespült wurde, wird die Reinigungsvorrichtung um 180° in Schwerkraftrichtung geschwenkt und nimmt die zweite Betriebsposition ein. In dieser zweiten Betriebsposition wird Luft unter Einfluss der Dosierpumpe vom Behälter durch die Reinigungsvorrichtung und den Instrumentenhohlraum gezogen und tritt aus einer Öffnung des Instrumentenhohlraums aus. Dabei wird Luft durch den Instrumentenhohlraum gespült. Diese Luft reist die sich im Instrumentenhohlraum befindende Flüssigkeit mit sich und entfernt auf diese Weise Flüssigkeit aus dem Instrumentenhohlraum. Zum Unterdruckausgleich bei Dispersion von Flüssigkeit oder Luft, wird Luft aus der Umgebung in den Behälter angesaugt. Das erfindungsgemässe Verfahren zur Reinigung eines medizinischen Instruments verwendet eine zuvor beschriebene erfindungsgemässe Reinigungsvorrichtung.

Beim erfindungsgemässen Verfahren wird das medizinische Instrument mittels eines erste Befestigungsmoduls an der Reinigungsvorrichtung befestigt. Mittels eines zweiten Befestigungsmoduls wird ein Aufnahmeelement an der Reinigungsvorrichtung befestigt. In dem Aufnahmeelement befindet sich eine Flüssigkeit mittels der der Instrumentenhohlraum gereinigt wird. Die Reinigungsvorrichtung weist einen Reinigungsvorrichtungshohlraum auf, der so ausgestaltet ist, dass bei befestigtem medizinischem Instrument und befestigtem Behälter die Flüssigkeit und/oder die Luft vom Innern des Behälters durch den Reinigungsvorrichtungshohlraum in den Instrumentenhohlraum gespült wird. Die Reinigungsvorrichtung weist dabei eine erste und eine zweite Betriebsposition auf. In der ersten Betriebsposition wird die Flüssigkeit aus dem Behälter durch den Reinigungsvorrichtungshohlraum in den Instrumentenhohlraum gespült und in der zweiten Betriebsposition wird Luft aus der Umgebung durch den Behälter und den Reinigungsvorrichtungshohlraum in den Instrumentenhohlraum gespült. Auf diese Weise wird im ersten Schritt der Instrumentenhohlraum mittels der durchströmenden Flüssigkeit gereinigt und im zweiten Schritt wird der Instrumentenhohlraum mittels der durchströmenden Luft getrocknet.

Da diese Verfahren reine Flüssigkeit in das Instrument einbringen, und nicht ein Luwflüssigkeit Gemisch ist eine lückenlose Benetzung alle Oberflächen garantiert.

Auf diese Weise findet die Reinigung und die anschliessende Luftspüllung ohne Ankupplung eines medizinischem Instrument an die Reinigungsvorrichtung statt, was die Arbeit vereinfacht.

Diese Verfahren sind vorteilhaft, da sie für das Durchspülen des medizinischen Instruments mit Flüssigkeit oder Gas kein unter Druck stehendes Gas benötigt wird. Dadurch sind auch keine teuren Verbindungen zu Druckgasleitungen notwendig. Durch das Nichtverwenden von Druckgas wird die Bildung von Spray und insbesondere kleine Tröpfchen von Reinigungsflüssigkeiten oder Ablagerungen im medizinischen Instrument vermieden. Dies stellt einen besonderen Vorteil dar, da durch das Einatmen solcher Sprays oder Tröpfchens zu Vergiftung, Irritationen oder Infekten beim Reinigungspersonal kommen kann. Ausserdem weist die Verwendung von Druckgas den zusätzlichen Nachteil auf, dass eine lückenlose Benetzung aller Oberflächen nicht garantiert werden kann.

Einen zusätzlichen Vorteil dieses Verfahrens weist die Möglichkeit des Füllens des medizinischen Instruments mit Flüssigkeit auf ohne dass Unterdruck oder ein Vakuum angelegt werden muss. Dadurch ist der teure und technisch aufwändige Aufbau eines Vakuums nicht notwendig. Ein zusätzlicher Nachteil beim Benutzen von Vakuum ist dass eine lückenlose Benetzung aller Oberflächen nicht sicher gestellt werden kann. Auch hier tritt das Problem der Tropfenbildung und die damit verbundenen Nachteile auf.

Ein weiterer Vorteil des Verfahrens besteht darin, dass im Innern des Behälters keine erhebliche Erhöhung des Drucks stattfindet. Dadurch sind keine besonderen Massnahmen notwendig, die bei unter Druck stehenden Bauteilen notwendig wären. Unter Druck stehende Bauteile bergen ausserdem die Gefahr, dass sie explodieren und dadurch Flüssigkeit und Gas im Raum verteilt werden, die eine Gefahr für das Reinigungspersonal darstellen.

Idealerweise erfolgt die Befestigung an das erste Befestigungsmodul mit Hilfe eines Adapters. Die Verwendung von Adaptern ermöglicht die Befestigung verschiedener zahnärztlichen und medizinischen Instrumenten mit unterschiedlichen Öffnungskonfigurationen an der Reinigungsvorrichtung. Von Vorteil ist es auch den Adapter möglichst einfach auszugestalten und zum einmaligen Gebrauch vorzusehen. Der einmalige Gebrauch des Adapters bietet den Vorteil, dass der Adapter nach jedem Gebrauch ersetzt wird und so die Hygienesicherheit auf günstige und einfache Art und Weise erheblich erhöht werden kann.

Idealweise, erfolgt die Befestigung vom Adapter an die Reinigungsvorrichtung durch eine standardisierte Verbindung, wie zum Beispiel einer normierten Luer-Lock Verbindung. Diese ermöglicht auch die Befestigung von weiteren Vorrichtungen zwischen Adapter und Reinigungsvorrichtung, vorausgesetzt diese weiteren Vorrichtungen verfügen auch über eine standardisierte Verbindung, wie beispielsweise einer Luer-Lock Verbindung. Eine mögliche Vorrichtung ist beispielsweise ein Filter, der zwischen Adapter und Reinigungsvorrichtung befestigt ist, wobei der Filter der Reinigung der Flüssigkeit vor Eintritt in den Instrumentenhohlraum dient. Selbstverständlicherweise können auch andere standardisierte Verbindungen verwendet werden.

Die Verbindungstelle des Adapters kann direkt an die Reinigungsvorrichtung angeschlossen werden. Alternativ kann die Verbindungstelle des Adapter mit der Reinigungsvorrichtung über einen Schlauch verbunden werden.

Sollen die medizinische oder zahnärztlichen Instrumente bewegliche oder rotierende Teile aufweisen, ist es von Vorteil, wenn solche Teile während der Reinigung, Desinfektion und/oder Pflege betätigt bzw. rotiert werden, da durch diese Betätigung bzw. Rotation sichergestellt ist, dass alle Oberflächen in Kontakt mit der Flüssigkeit kommen. Es ist nicht notwendig, diese Betätigung in einer solchen Weise durchzuführen, dass die volle Funktion des medizinischen oder zahnärztlichen Instruments während der Reinigung erreicht wird. Es reicht aus wenn die beweglichen Teile mehrmals den vollen Bewegungs- bzw. Rotationszyklus durchlaufen. Es reicht beispielweise aus, wenn die beweglichen Teile eines Handstückes durch einen Antrieb mehrmals rotiert werden. Diese Rotation kann manuell oder motorbetrieben ausgeführt werden.

Die verwendete Flüssigkeit ist idealerweise eine Reinigungsflüssigkeit die vorzugsweise ein Desinfektionsmittel aufweist. Das Desinfektionsmittel ist derart gewählt, dass beim Reinigen der Instrumentenhohlraum desinfiziert wird.

Von einer viruswirksamen Desinfektion wird gesprochen, wenn beispielsweise das Desinfektionsmittel innerhalb definierter Einwirkzeiten in der Lage ist, den Virustiter (entspricht der Menge der in einem Zellkulturlysat pro Volumeneinheit vorhandenen infektiösen Viren) der Prüfvirenstämme um mindestens 4 dezimallogarithmische Stufen bzw. 4 Log-Stufen zu verringern. Eine Reduktion um eine Log-Stufe entspricht einer zehnfachen bzw. 90 %-igen Reduktion der Virenstämme. Entsprechend entspricht eine Reduktion um 4 Log-Stufen einer 10<4>-fachen bzw. 99.99 %-igen Reduktion der Virenstämme. Sind anfänglich beispielsweise 10<6> Virenstämmen vorliegend, überleben bei einer Reduktion um 4 LogStufen lediglich 10<2> Virenstämme.

Mit Vorteil werden daher sogenannte viruzide Desinfektionsmittel eingesetzt, welche sowohl gegen behüllte Viren als auch gegen unbehüllte Viren wirksam sind. Im Allgemeinen schliesst dabei bei alkoholischen Desinfektionsmitteln eine Wirksamkeit gegen unbehüllte Viren auch eine Wirksamkeit gegen sämtliche behüllten Viren mit ein. Gemäß der Norm EN 14476 (aus dem Jahre 2005) gilt ein Desinfektionsmittel als viruzid, wenn es in der Lage ist, die folgenden unbehüllten Virenstämme unter definierten Testbedingungen zu inaktivieren:
- Poliovirus Typ 1, LSc - 2ab
- Adenovirus Typ 5, Stamm Adenoid 75, ATCC VR-5
Die Leitlinie der Deutschen Vereinigung zur Bekämpfung der Viruskrankheiten (DW) e.V. und des Robert Koch-Institut (RKI) in der Fassung aus dem Jahr 2005 (DW/RKI-Leitlinie) fordert für viruzide Desinfektionsmittel ein erweitertes Testverfahren, bei welchem zusätzliche unbehüllte als auch behüllte Virenstämme berücksichtigt werden:
- Vacciniavirus, Stamm Elstree
- Poliovirus Typ 1 , LSc - 2ab - Adenovirus Typ 5, Stamm Adenoid 75
- Polyomavirus (SV 40), Stamm 777

Während der Reinigung ist das medizinische Instrument nicht im Einsatz am Patienten. Es ist deshalb die Verwendung einer Reinigungsflüssigkeit die reizend oder korrosiv auf lebendes Gewerbe wirkt möglich und sinnvoll, vorausgesetzt diese Flüssigkeit wird nach Abschluss des Reinigungsverfahrens vollständig aus dem medizinischen Instrument entfernt.

Bevorzugterweise wird zur Reinigung eine Flüssigkeit verwendet, die mehrere Komponenten aufweist. Diese Komponenten können eine homogene Mischung aufweisen. Es kann jedoch vorteilhaft sein eine inhomogene Mischung zu verwenden.

Idealerweise ist eine der Komponenten Öl. Diese Ausgestaltung ist besonders vorteilhaft, da dabei beim Reinigungsvorgang bereits Öl in den Instrumentenhohlraum gebracht wird. Ein Teil des Öls verbleibt auch nach dem Reinigungsvorgang im Instrumentenhohlraum. Auf diese Weise wird der Instrumentenhohlraum bereits während des Reinigungsvorgangs geölt. Der zusätzliche Schritt des Anschliessenden Ölens des Reinigungshohlraums entfällt und vereinfacht dadurch den Reinigungsvorgang. Der Anwender muss ausserdem keine zusätzliche Flüssigkeit zum Ölen bevorraten. Was ebenfalls einen Vorteil darstellt. Besonders Vorteilhaft ist die Präsenz von Öl bei alkoholhaltigen Reinigungsflüssigkeiten, da ölfreie alkoholhaltige Flüssigkeiten das an dem medizinischen Instrument notwendig und vorhandene Öl vollständig entfernen. Wird nach einer Reinigung mit einer ölfreien, alkoholbasiertem Flüssigkeit versehendlich die anschliessendes Ölung vergessen, kann es bei rotierenden Teilen wie beispielsweise zahnärztlichen Turbinen die mit bis zu 500 000 rpm Rotieren schnell zur grossen Schaden kommen. Die Kombination der erfindungsgemässen Reinigungsvorrichtung mit einer ölhaltigen Flüssigkeit stellt somit einen wesentlichen Vorteil für den Benutzer dar.

Der Volumenanteil des Öls in der Flüssigkeit ist idealerweise grösser als 0.2 % und kleiner als 10 %. Besonders vorteilhaft ist ein Volumenanteil von 0.3% bis 0.7%. Die Verwendung von Hochleistungsdentalöl hat besonders gute Ergebnisse gezeigt.

Idealerweise besteht eine der Komponenten aus kurzkettige Alkoholen und der Volumenanteil der kurzkettigen Alkohole in der Flüssigkeit ist grösser als 40 % und kleiner als 80 %. Besonders gute Reinigungsergebnisse haben sich für einen Volumenanteil der kurzkettigen Alkohole in der Flüssigkeit von grösser als 60 % und kleiner als 75 % gezeigt. Lösungen mit einem solchem Anteil an kurzkettigen Alkoholen haben geringe Korrosivität auf Eisen.

Idealerweise weist die Flüssigkeit anorganische Säuren auf, da derartige Säuren die antimikrobielle Wirksamkeit erhöhen und die Kalkablagerungen in wasserführenden Kanälen entfernen. Beispiele für solche Säuren sind unter anderem Salzsäure, Schwefelsäure, Amidosulfonsäure, Phosphorsäure.

Idealerweise weist die Flüssigkeit Phosphorsäure auf und der Volumenanteil des Phosphorsäure in der Flüssigkeit ist grösser als 0.1 % und kleiner als 2 %. Besonders gute Ergebnisse haben sich für einen Volumenanteil der Phosphorsäure in der Flüssigkeit von grösser als 0.7 % und kleiner als 1 % gezeigt. Phosphorsäure weisst zusätzliche antikorrosive Eigenschaften auf.

Idealerweise ist die verwendete Flüssigkeit ein Desinfektionsmittel, das gegen alle Viren, alle Bakterien und Hefepilzen wirksam ist.

Es sind jedoch auch andere Reinigungsflüssigkeiten möglich. So kann zum Beispiel eine Reinigungslösung auf Basis von Wasser und Tensiden verwendet werden. Solche Reinigungsflüssigkeiten könnten auch Zusatzkomponenten enthalten, die einen zusätzlichen Vorteil bringen. Eine mögliche Zusatzkomponente sind beispielsweise Enzyme, die die Proteinentfernung unterstützen. Von Vorteil ist auch die Verwendung von bereits bekannten und anerkannten Reinigungsflüssigkeiten, wie sie beispielsweise nach der internationalen Norm 15883 getestet sind. Möglich sind auch Desinfektionsmittel die auf wässrigen Lösungen von Biozide basieren, wie beispielweise Alkohole, Quaternäre Ammonium Verbindungen, Alkylamin - Derivaten, Guanidin-Derivaten, Aldehyden, Halogene, Peroxidverbindungen, Phenolen und andere. Ebenso möglich sind Pflegeflüssigkeiten zum regelmässigen Gebrauch wie beispielsweise Schmiermittel, die auf einer homogene Mischung von kurzkettigen Alkoholen und Öl oder einer stabile Suspension eines Öls in wässriger Flüssigkeit basieren. Ebenso möglich sind Pflegeflüssigkeiten zum einmaligen oder seltenen Gebrauch, wie eine Lösung von Fluorotensiden zur einmaligen Beschichtung der inneren Oberflächen des medizinischen Instruments. Diese Flüssigkeiten können beispielsweise als gebrauchsfertige Lösungen oder als Konzentrate zur Verdünnung mit Wasser dem Anwender zur Verfügung gestellt werden. Es ist auch möglich ein mehrkomponentiges Set zur Herstellung der Flüssigkeit vor der Anwendung dem Anwender zur Verfügung zu stellen.

Idealerweise erreicht die Reinigung, Desinfektion oder Pflegeflüssigkeit seine Wirkung innerhalb von kurzer Zeit. Vorzugsweise ist die Wirkung innerhalb einer Minute erreicht. Bei längeren Einwirkungszeiten besteht die Gefahr, dass der Reinigungsvorgang vor Wirkungsantritt abgebrochen wird. Zusätzlich steigt bei längeren Einwirkungszeiten die Gefahr dass die Materialien von medizinischen Instrumenten vom Reinigungsmittel angegriffen werden.

Idealerweise enthält die Reinigungs-, Desinfektions- und/oder Pflegeflüssigkeit keine Komponenten die toxisch für Menschen sind und korrosiv auf medizinische Instrumenten wirken in einer Konzentration die 0.1% überschreitet. Solche unerwünschte Komponente sind beispielsweise Salze von Schwermetallen, wie beispielsweise Quecksilberverbindungen.

Idealerweise wird für die Reinigung, Desinfektion oder Pflege eine Flüssigkeit verwendet, die für innere sowie äussere Anwendung am medizinischen Instrument geeignet ist.

Ein Vorteil des Verfahrens ist es, dass die Reinigung, Desinfektion oder Pflege bei Raumtemperatur durchgeführt werden kann. Als Raumtemperatur wird eine Temperatur von 15 - 25°C verstanden. Die Verfahren kann jedoch auch problemlos Temperaturen von 5 - 40°C durchgeführt werden.

Aus dem Stand der Technik sind Verfahren bekannt, die wesentlich höhere Temperaturen benötigen. Üblicherweise werden diese Verfahren bei Temperaturen von 60 - 135 °C durchgeführt. Diese hohen Temperaturen führen zu einer Reihe von Problemen. So werden die gereinigten Instrumente stärker beansprucht, was Ermüdungsbrüche und/oder Ermüdungskorrosion fördert, was die Lebensdauern der teuren medizinische und zahnärztliche Instrumente verkürzt. Ausserdem brauchen thermische Verfahren spezielle, teure Geräte wie beispielsweise einen Autoklav. Des Weiteren sind thermische Verfahren zeitintensiver, da bis zum Abkühlen des Instruments eine längere Wartezeit in Kauf genommen werden muss.

Die erfindungsgemässe Vorrichtung wird anhand von Ausführungsformen, wie sie schematisch in den Zeichnungen dargestellt sind, im Folgenden beschrieben. Es zeigen
- Figur 1: einen Querschnitt durch eine erste Ausführungsform der erfindungsgemässen Reinigungsvorrichtung mit befestigtem Behälter und medizinischem Instrument in der zweiten Betriebsposition;
- Figur 2: einen Querschnitt durch eine zweite Ausführungsform der erfindungsgemässen Reinigungsvorrichtung mit befestigtem Behälter und medizinischem Instrument in der zweiten Betriebsposition;
- Figur 3a: einen Querschnitt durch die erste Ausführungsform der erfindungsgemässen Reinigungsvorrichtung mit befestigtem Behälter in der ersten Betriebsposition;
- Figur 3b: einen Querschnitt durch die erste Ausführungsform der erfindungsgemässen Reinigungsvorrichtung mit befestigtem Behälter in der ersten Betriebsposition während des Pumpvorgangs;
- Figur 3c: einen Querschnitt durch die erste Ausführungsform der erfindungsgemässen Reinigungsvorrichtung mit befestigtem Behälter in der ersten Betriebsposition;
- Figur 3d: einen Querschnitt durch die erste Ausführungsform der erfindungsgemässen Reinigungsvorrichtung mit befestigtem Behälter in der ersten Betriebsposition in Nachfüllmodus;

### Beschreibung der Figuren

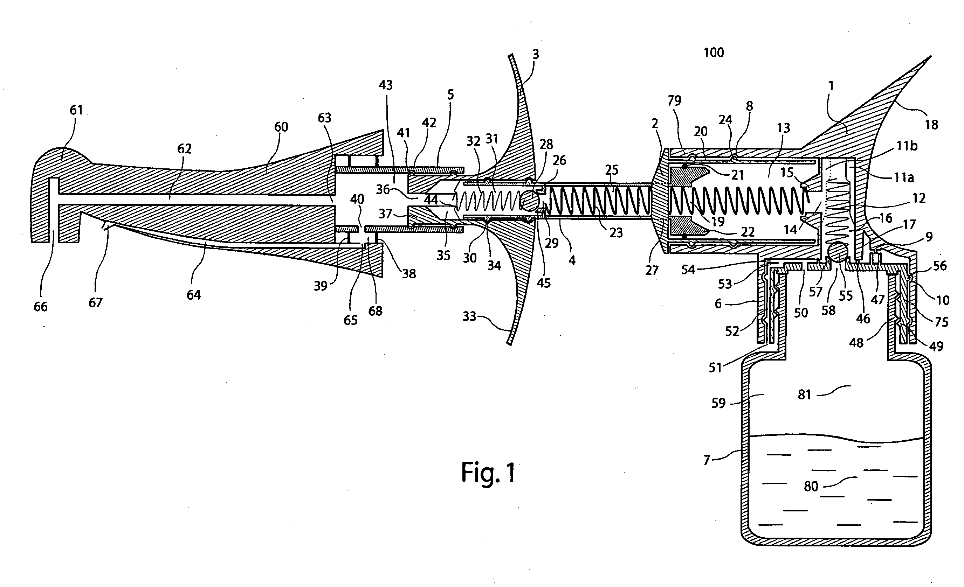

Figur 1 zeigt einen Querschnitt durch eine erste Ausführungsform der erfindungsgemässen Reinigungsvorrichtung 100 mit befestigtem Behälter 7 und medizinischem Instrument 60 in der zweiten Betriebsposition. Der Behälter 7 dient der Aufnahme von Flüssigkeiten 80 zur Reinigung des medizinischen Instruments 60. Die Reinigungsvorrichtung 100 ist derart ausgestaltet, dass Flüssigkeit oder Gas vom Innern des Behälters durch die Reinigungsvorrichtung in den Instrumentenhohlraum 62 des medizinischen Instruments 60 gebracht werden kann. Die Reinigungsvorrichtung 100 weist ein zweites Befestigungsmodul 6 zur lösbaren Befestigung des Behälters 7 auf. Das zweite Befestigungsmodul 6 weist ein aus der Reinigungsvorrichtung 100 reversible entfernbares Gewindeeinsatz 75 auf. Dieser reversibel entfernbare Gewindeeinsatz ist zylinderförmig und weist an der Innenseite einen Gewinde 49 auf. Ein Ende dieses Teils ist offen und dient der Aufnahme des Behälters 7. Das andere Ende dieses Teils ist bis auf Öffnung 58 zum Durchlass von Flüssigkeit vom Behälter 7 in die Reinigungsvorrichtung 100 und einer Auslassöffnung 50 für Umgebungsluft des Lufteinlasssystem in den Behälter 7 geschlossen. An der Aussenseite weist es Vorrichtungen zur reversiblen Entfernung und Befestigung im Befestigungsmodul 6 auf. Diese Vorrichtung zur reversible Entfernung und Befestigung ist bei der hier dargestellten Ausführungsform eine zusammenwirkende Sicherungsnut 10 und ein Sicherungsüberstand 56. Es sind jedoch auch andere Ausführungsformen der reversiblen Befestigung und Entfernung möglich. Beispielsweise kann eine zusammenwirkende Schraubverbindung oder eine Nut und Feder Verbindung verwendet werden. Das reversible entfernbare Teil des Befestigungsmoduls 6 hat den Vorteil, dass einfach ein anderes Teil mit beispielsweise einem anderen Gewindedurchmesser eingesetzt werden kann. Dies bietet den Vorteil, dass auf die Reinigungsvorrichtung 100 für verschiedene Behälter verwendet werden kann, bzw. auf einfache und schnelle Weise umgerüstet werden kann. Um sicherzustellen, dass das reversibel entfernbare Teil des Befestigungsmodul 6 korrekt positioniert ist, sind an diesem Teil und der Reinigungsvorrichtung eine zusammenwirkender Positionierungsüberstand 9 und Positionierungskerbe 47 angeordnet. Der Behälter 7 ist bei dieser Ausführungsform eine Flasche mit einem angeformten Flaschengewinde 48. Das Flaschengewinde 48 wirkt mit einem Gewinde 49 des Gewindeeinsatzes 75 des zweiten Befestigungsmoduls 6 zusammen. Die Achse des Gewindes 49 des Gewindeeinsatzes 75 und des Flaschengewindes 48 sind sowohl in der ersten und als auch der zweiten Betriebsposition vertikal ausgerichtet. Fliesst die Flüssigkeit während des Reinigungsvorgangs aus dem Behälter 7, so entsteht dabei ein Unterdruck. Um Luft in den Behälter 7 einlassen und den Unterdruck auszugleichen, weist das zweite Befestigungsmodul 6 einen Luftkanal 52 auf mittels dem Umgebungsluft in das Innere des Behälters 7 gebracht werden kann. Der Luftkanal 52 muss dabei so ausgestaltet sein, dass die Umgebungsluft zwar in den Behälter 7 gelangen kann. Die Flüssigkeit jedoch nicht vom Behälter 7 durch den Luftkanal 52 in die Umgebung austreten kann. Um dieses Problem zu lösen ist der Luftkanal 52 derart ausgestaltet, dass der Luftkanal 52 in der zweiten Betriebsposition sich seine Einlassöffnung 51 an der Unterseite des zweiten Befestigungsmoduls 6 befindet. Der Luftkanal 52 verläuft mehrheitlich in vertikaler Richtung im Befestigungsmodul 6. Um den Flüssigkeitsaustritt zu erschweren ist am Luftkanal ein Raum 54 angeordnet, in dem eventuell austretende Flüssigkeit gesammelt würde. Ausserdem weist der Luftkanal 52 einen horizontalen Kanalabschnitt 53 aus. Die Kombination aus horizontalem und vertikalem Kanalabschnitt bildet eine Art Labyrinth, das es der Flüssigkeit erschwert in die Umgebung auszutreten.

Innerhalb der Reinigungsvorrichtung 100 ist bei der Öffnung zum Flüssigkeitsdurchlass 58 zwischen Behälter 7 und Reinigungsvorrichtung 100 auf der Innenseite ein Einlassventil angeordnet. Das Einlassventil ist in einem Einlassventilzylinder 17 angeordnet, der sich in der zweiten Betriebsposition in vertikaler Richtung über der Öffnung 58 erstreckt. Das Einlassventil weist eine Einlassventilkugel 55 und eine Einlassventilfeder 12 auf. Die Einlassventilfeder 12 steht unter Spannung. Die Einlassventilfeder 12 weist zwei Enden auf, wobei das eine Ende auf einer Stützplatte 11a, 11 b und das andere Ende auf der Einlassventilkugel 55 ruht. Die Einlassventilkugel 55 ist derart ausgestaltet, dass sie auf Grund der Federkraft der Einlassfeder 12 auf einer um die Öffnung 58 angeordnete Stützbühne 57 gehalten wird. Der Einlassventilzylinder 17 weist eine sich vertikal erstreckende Einlassöffnung 14 auf, in einen Zylinder 2 auf. Durch diese Einlassöffnung 14 kann Flüssigkeit aus dem Einlassventilzylinder 17 in den Zylinder 2 fliessen. Die Stützbühne 57 ist an das reversible entfernbare Teil des Befestigungsmoduls 6 angeformt. Um einen Flüssigkeitsaustritt bei der Öffnung 58 zu unterbinden ist um die Stützbühne 57 ein Dichtungsring 46 angeordnet. Die Reinigungsvorrichtung 100 weist ein Zylindergehäuse 1 auf. Die Achse des Zylindergehäuse 1 ist sowohl in der ersten als auch in der zweiten Betriebsposition horizontal zur Schwerkraftrichtung ausgerichtet. Durch Rotation der Reinigungsvorrichtung 100 um ca. 180° um die Achse des Zylindergehäuses 1 wird zwischen erster und zweiter Betriebsposition gewechselt. Im Innern des Zylindergehäuses 1 ist ein Zylinder 2 angeordnet. Die Achse des Zylinders 2 ist in der zweiten Betriebsposition horizontal zur Schwerkraftrichtung ausgerichtet. Der Zylinder 2 ist im Zylindergehäuse 1 fixiert. Bei dieser Ausführungsform weist der Zylinder 2 einen Sicherungsüberstand 24 auf, der mit einer an das Zylindergehäuse 1 angeformte Sicherungskerbe 8 zusammenwirkt. Der Zylinder 2 weist zwei sich gegenüberliegende Stirnseiten auf. Die erste Stirnseite weist eine Kolbenöffnung 27 auf. In dieser Kolbenöffnung 27 ist ein Hohlkolben 4 horizontal beweglich gelagert. Der Hohlkolben 4 weist ein erstes und ein zweites Ende auf. An das erste Ende des Hohlkolbens 4 ist ein Hohlkolbenkopf 22 angeformt. Dieser Hohlkolbenkopf 22 ist im Innern des Zylinders 2 dem sogenannten Zylinderraum 13 angeordnet. Der Hohlkolbenkopf 22 ist derart ausgestaltet, dass er mit der Innenwand 20 des Zylinders 2 derart zusammenwirkt, dass möglichst wenig Flüssigkeit zwischen Hohlkolbenkopf 22 und Innenwand 20 austreten. Um einen Durchgang von Flüssigkeit zusätzlich zu erschweren ist zwischen Hohlkolbenkopf 22 und Innenwand 20 eine Dichtung 21 angeordnet. Das zweite Ende des Hohlkolbens 4 ist über einen Adapter 5 mit dem Instrumentenhohlraum 62 des medizinischen Instruments 60 verbunden. Im Innern des Hohlkolbens 4 ist eine Hohlkolbenfeder 23 angeordnet, die in horizontaler Richtung gespannt ist. Die Hohlkolbenfeder 23 weist ein erstes und ein zweites Ende auf. Das erste Ende ruht auf einer am Zylindergehäuse 1 angeformten ersten Stützbühne 15. Das zweite Ende ruht auf einer im Hohlkolbeninnenraum 25 angeordneten zweiten Stützbühne 26 der Hohlkolbenfeder 23. Die zweite Stützbühne 26 teilt vom Hohlkolbeninnenraum 25 einen Auslassventilraum 31 ab. Die zweite Stützbühne 26 weist eine Auslassventilöffnung 29 auf durch die Flüssigkeit hindurchfliessen kann. Im Auslassventilraum 31 ist ein Auslassventil angeordnet, das eine Auslassventilfeder 32 und eine Auslassventilkugel 28 aufweist. Die Auslassventilkugel 28 ist derart vor der Auslassventilöffnung 29 angeordnet, dass ein unkontrolliertes Austreten von Flüssigkeit vermieden wird. Zur Positionierung der Auslassventilkugel 28 ist der Stützbuhne 26 eine zusätzliche Stützbühne 45 für die Auslassventilkugel 28 angeformt. Die Auslassventilfeder 32 ist in horizontaler Richtung gespannt und weist ein erstes und ein zweites Ende auf. Das erste Ende der Auslassventilfeder 32 ist bei der Auslassventilkugel 28 angeordnet. Das zweite Ende der Auslassventilfeder 32 ist einer Auslassventilfederstützplatte 44 abgestützt. Die Auslassventilfederstützplatte 44 kann auf unterschiedliche Weise realisiert werden. Es ist beispielsweise möglich sie direkt im Hohlkolben 4 anzuformen. Bei dieser Ausführungsform wurde hingegen zwischen Hohlkolben 4 und Adapter 5 ein Kolbengriff 3 angeordnet. An den Kolbengriff 3 ist ein anatomisch geformter Bogenlauf 33 angeformt. Mit Hilfe des Kolbengriffs 3 kann der Hohlkolben 4 horizontal verschoben werden. Diese horizontale Verschiebung dient dem Flüssigkeits- bzw. Gastransports in der Reinigungsvorrichtung 100. An das Zylindergehäuse 1 kann ebenfalls ein anatomisch geformter Bogenlauf 18 angeformt sein. Die anatomisch geformten Bogenläufe 33, 18 ermöglichen eine komfortable Handhabung der Reinigungsvorrichtung 100. An den Kolbengriff 3 ist die Auslassventilfederstützplatte 44 angeformt. Hohlkolben 4, Kolbengriff 3 und Adapter 5 sind fest miteinander verbunden. Dazu sind an den Holkolben 4 Sicherheitsüberstände 30 angeformt, die mit Sicherheitskerben 34 des Kolbengriffs 3 zusammenwirken und an den Kolbengriff 3 sind Sicherheitsüberstände 41 angeformt, die mit Sicherheitskerben 42 des Adapters 5 zusammenwirken. Es wäre auch denkbar Hohlkolben 4, Kolbengriff 3 und Adapter 5 auch aus einem einzigen Stück zu formen. Es wäre auch denkbar Kolbengriff 3 und Adapter 5 aus einem Stück zu formen. Es wäre auch denkbar das zweite Befestigungsmodul 6 ebenfalls als einen integralen Teil des Zylindergehäuses 1 zu formen.

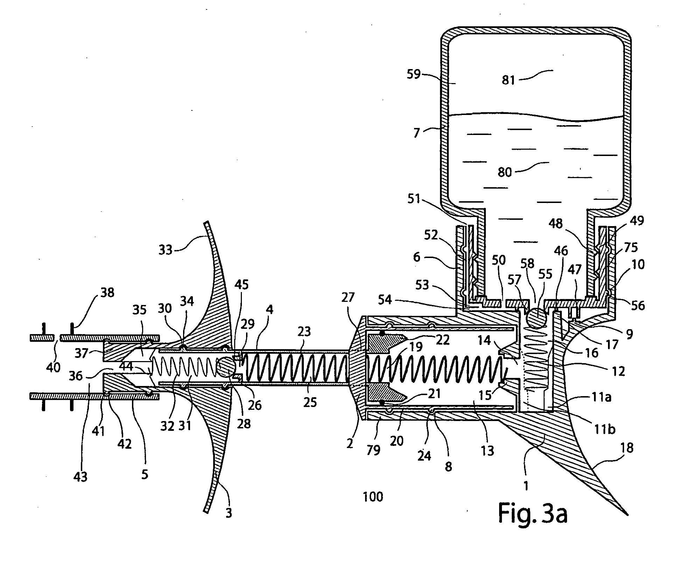

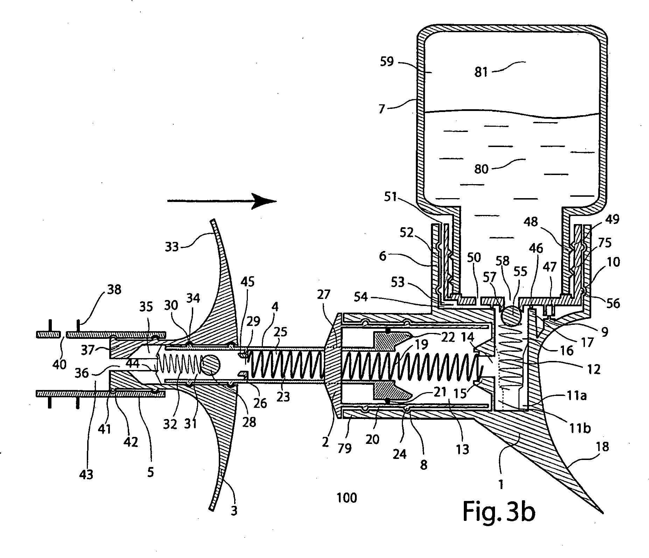

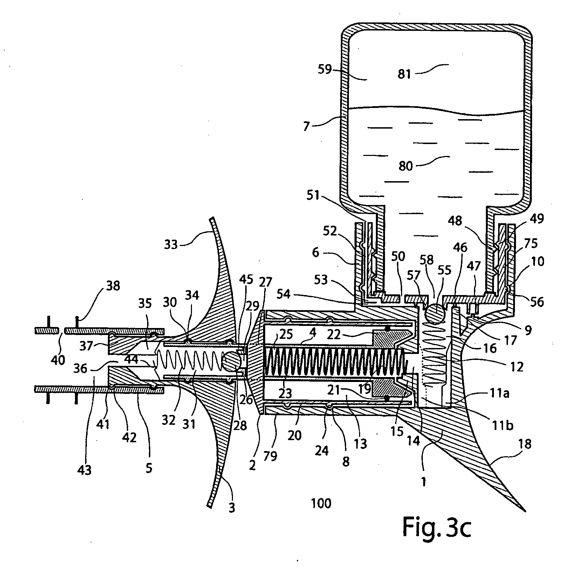

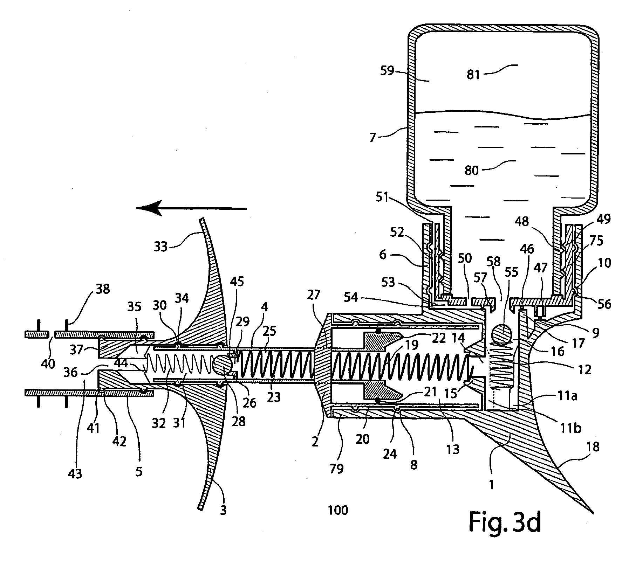

Die Auslassventilfederstützplatte 44 weist eine Auslassöffnung 36 auf durch die Flüssigkeit durchfliessen kann. Diese Auslassöffnung 36 ist derart ausgestaltet, dass sie eine Düse 37 bildet. Die Düse 37 weist einen sich verengenden Querschnitt auf, der die Aufgabe hat den Flüssigkeitsstrahl zu bündeln, damit die Flüssigkeit mit einer grösseren Geschwindigkeit in das medizinische Instrument 60 gespült werden kann. Die höhere Geschwindigkeit wirkt sich positiv auf das Reinigungsverhalten der Reinigungsvorrichtung 100 aus. Der Adapter 5 dient der Verbindung zwischen Reinigungsvorrichtung 100 und medizinischen Instrument 60. Der Adapter 5 ist für die Reinigung eines bestimmten medizinischen Instruments 60 ausgelegt. Soll mit der Reinigungsvorrichtung 100 ein anderes medizinisches Instrument 60 gereinigt werden muss dazu lediglich der Adapter 5 ausgewechselt werden. Es ist deshalb vorteilhaft die den Adapter 5 mittels einer Steckverbindung mit den restlichen Teilen der Reinigungsvorrichtung 100 zu verbinden. Bei der gezeigten Ausführungsform ist der Adapter 5 mittels einer Nutverbindung mit dem Kolbengriff 3 verbunden. Soll der Adapter 5 gewechselt werden, kann er einfach durch eine Ziehbewegung vom Kolbengriff 3 entfernt werden. Ein anderer Adapter kann danach mit einer einfachen Druckbewegung auf dem Kolbengriff 3 fixiert werden. Danach kann die Reinigungsvorrichtung sofort für die Reinigung eines anderen medizinischen Instruments verwendet werden. Das in Figur 3 dargestellte medizinische Instrument 60 weist einen Arbeitskopf 61 auf. Im medizinischen Instrument 60 ist ein Instrumentenhohlraum 62, der sogenannte Hauptkanal, angeordnet. Ausserdem ist im medizinischen Instrument 60 ein weiterer Instrumentenhohlraum, ein sogenannter Nebenkanal 64, angeordnet. Der Hauptkanal 62 weist eine Einlassöffnung 63 und eine Auslassöffnung 66 auf. Die Einlassöffnung 63 des Hauptkanals 62 befindet sich bei befestigter Reinigungsvorrichtung 100 in der Verlängerung der Achse des Hohlkolbens 4. Bei dieser Ausgestaltung trifft der aus der Düse 37 tretende Wasserstrahl direkt auf die Einlassöffnung 63, was eine optimale Reinigung des Hauptkanals 62 sicherstellt. Der Nebenkanal 64 weist eine Einlassöffnung 65 und eine Auslassöffnung 67 auf. Die Einlassöffnung 65 ist in axialer Richtung angeordnet und bei befestigter Reinigungsvorrichtung 100 ist so von der Verlängerung der Achse des Hohlkolbens 4 beabstandet. Für die Reinigung dieser Ausgestaltung des medizinischen Instruments 60 wird ein zylinderförmiger Adapter 5 mit offenen Enden verwendet. Die Achse des Adapters 5 stimmt mit der Achse des Hohlkolbens 4 überein. Das eine Ende des Zylinders ist mit Hilfe eine Nutverbindung 41, 42 mit dem Kolbengriff 3 verbunden, das andere Ende des Zylinders schliesst sich formbündig an das medizinische Instrument 60. Zur Reinigung des Nebenkanals 64 weist der Adapter 5 eine seitliche Öffnung 40 im Zylinder auf. Um ein Austreten der Flüssigkeit zu verhindern ist vor der seitlichen Öffnung 40 ein erster Dichtring 38 und nach der seitlichen Öffnung 40 ein zweiter Dichtring 39 angeordnet. Der Raum zwischen dem ersten und zweiten Dichtring wird als Innenraum zwischen erster und zweiter Dichtung bezeichnet 68. Der Raum innerhalb des Adapters 5 wird mit Adapterraum 43 bezeichnet.

Das Einlassventil 12,17,55, das Auslassventil 31,32,55 und der Kolbenraum 4 bilden eine Dosierpumpe 79.

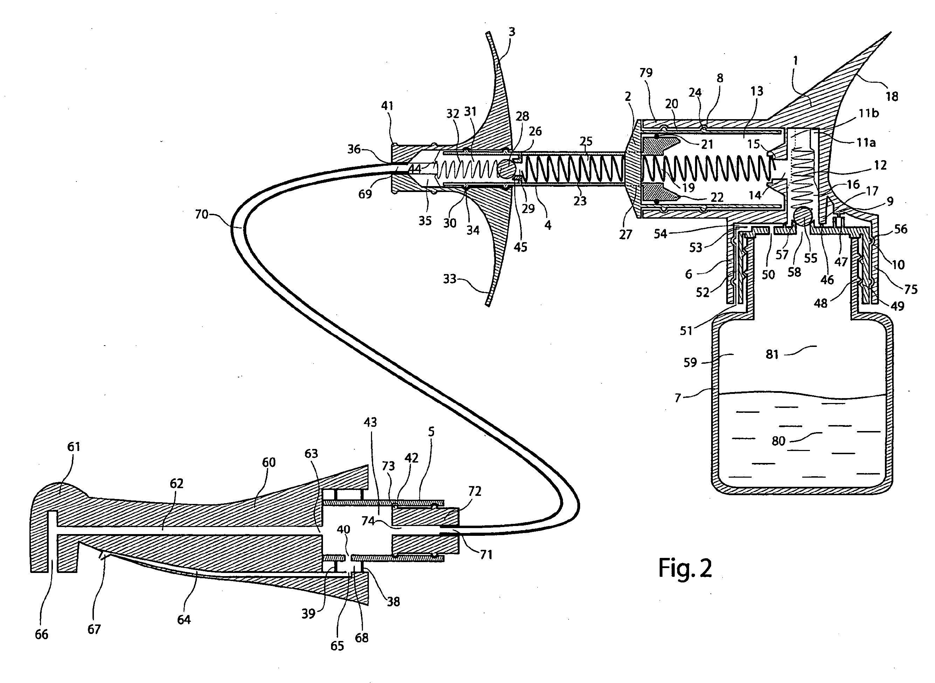

Figur 2 zeigt einen Querschnitt durch eine zweite Ausführungsform der erfindungsgemässen Reinigungsvorrichtung 100 mit befestigtem Behälter 7 und medizinischem Instrument 60 in der zweiten Betriebsposition dar. Diese Reinigungsvorrichtung 100 ist nahezu identisch mit der Ausführungsform in Figur 1. Lediglich Kolbengriff 3 und Adapter 5 sind über einen Schlauch 70 miteinander verbunden. Der Schlauch 70 weist zwei ein erstes Schlauchende 69 und ein zweites Schlauchende 71 auf. Das erste Schlauchende 69 ist in die Auslassöffnung 36 des Kolbengriffs 3 eingebracht. Das zweite Schlauchende 71 ist mit Hilfe Zwischenelements 72 mit dem Adapter 5 derart verbunden, dass Flüssigkeit aus dem Kolbengriff 3, durch den Schlauch 70 in den Adapter 5 fliessen kann. Das Zwischenelement 72 wirkt formschlüssig mit dem Adapter 5 zusammen, wodurch der Adapter 5 mittels einer Nutverbindung 73, 42 mit dem Zwischenelement verbunden ist. Bei dieser Ausgestaltung trifft der aus der Düse 74 tretende Wasserstrahl direkt auf die Einlassöffnung 63, was eine optimale Reinigung des Hauptkanals 62 sicherstellt.

Der Reinigungsvorgang wird Anhand der Figuren 3a, 3b, 3c und 3d beschrieben. Figur 3a zeigt die Reinigungsvorrichtung 100 betriebsbereit in der ersten Betriebsposition. In diese Position drückt die Einlassventilfeder 12 auf die Kugel des Einlassventils 55 und versperrt die Öffnung 58 des Einlassventils. Die Auslassventilfeder 32 drückt auf die Kugel des Auslassventils 28 und versperrt die Öffnung 29 des Auslassventils. Somit fliesst kein Fluid durch die Reinigungsvorrichtung.

Die Figur 3b zeigt die Reinigungsvorrichtung 100 in der ersten Betriebsposition in Dispensionsmodus. Der Hohlkolben 4 ist dabei in den Zylinder 2 eingeschoben zu werden. Dazu drück der Benutzer mit den Finger auf den anatomischen Bogenlauf 33 des Kolbengriffs 3 Dabei wird im Kolbenraum 13 Druck aufgebaut. Der Dichtungsring 21 verhindert dass das Fluid vom Kolbenraum 13 neben dem Hohlkolbenkopf 22 austreten kann. Das unter Druck stehende Fluid und die unter Druck stehende Einlassventilfeder 12 drücken die Kugel des Einlassventils 55 gegen die Öffnung 58 und verschliessen auf diese Weise das Einlassventil. Der Dichtungsring 46 verhindert dass das Fluid vom Einlassventilraum 16 in den Raum des Lufteinlasssystem 54 austritt. Somit wird das Fluid durch den Kolbenraum 13 und den Einlassventilraum 16 durch die Einlassöffnung Hohlkolben 19 gedrückt. Das Fluid fliesst durch den Innerraum des Hohlkolbens 25 und übt Druck auf die Kugel des Auslassventils 28 aus. Dieser Druck ist grösser als der Druck der Auslassventilfeder 32. Dadurch wird die Kugel des Auslassventils 28 von der Öffnung des Auslassventils 29 weggedrückt, was eine Öffnung des Auslassventil 29 bewirkt, wodurch das Fluid durch den Auslassventilraum 31, die Auslassöffnung 36 und den Raum des ersten Befestigungsmoduls 43 in den bzw. die Hohlräume das medizinisches Instruments fliessen kann. Je nach Art der verwendeten Flüssigkeit wird eine unterschiedliche Wirkung erreicht. Je nach Art der Befestigung werden Haupthohlraum 62 oder Nebenhohlraum 65 oder beide mit der Flüssigkeit gespült.

Figur 3c zeigt die Reinigungsvorrichtung 100 in der ersten Betriebsposition voll entleert. In diese Position drückt die Einlassventilfeder 12 auf die Kugel des Einlassventils 55 und versperrt die Öffnung 58 des Einlassventils. Die Auslassventilfeder 32 drückt auf die Kugel des Auslassventils 28 und versperrt die Öffnung 29 des Auslassventils 28. Somit fliesst kein Fluid durch die Reinigungsvorrichtung 100.

Figur 3d zeigt die Reinigungsvorrichtung 100 in der erste Betriebsposition in Nachfüllmodus, wobei der Hohlkolben 4 bereits zu 2/3 aus dem Zylinder 2 herausgezogen wurde. Unter Druck der Hohlkolbenfeder 23, wird der Hohlkolben 4 aus dem Zylinder 2 gedrückt. Auf diese Weise wird im Kolbenraum 13 ein Unterdruck aufgebaut. Der Dichtungsring 21 verhindert dass Luft in den Kolbenraum 13 neben dem Hohlkolbenkopf 22 einfliesst. Unter Unterdruck im Kolbenraum 13 und im Innerraum der Hohlkolben 25 sowie unter Druck der Auslassventilfeder 32 wird die Kugel des Auslassventils 28 an die Öffnung 29 des Auslassventils gepresst und verschliesst diese. So kann kein Fluid aus dem Instrument 60 angesaugt werden. Der Unterdruck auf die Kugel des Einlassventils 65 ist grösser als der Druck von der Einlassventilfeder 12. Somit wird die Kugel des Auslassventils 12 von der Öffnung Auslassventil 58 weggedrückt, wodurch die Öffnung des Auslassventils 58 geöffnet wird. Unter Unterdruck fliesst das Fluid durch den Einlassventilraum 16 aus den Behälter 7, und durch den Einlassöffnung Kolbenraum 14 in den Kolbenraum 13 und füllt diesen. Wenn der Hohlkolbenkopf 22 das Ende des Zylinders 2 erreicht hat, ist die Reinigungsvorrichtung erneut betriebsbereit und es kann wie in Figur 3a gezeigt ist fortgefahren werden.

Wiederholte Betätigung der Reinigungsvorrichtung 100 in der erste Betriebsposition in der in Figur 3a bis 3d gezeigten Sequenz bewirkt wiederholtes Pumpen von Flüssigkeit 80 aus dem Behälter 7 in den bzw. die Hohlräume 62, 64 des medizinischen Instruments 60.

In die Zweite Betriebsposition, sind die Schritte genau gleich, mit dem Unterschied dass mittels der Reinigungsvorrichtung Luft durch das medizinische Instrument bewegt wird und auf diese Weise die Hohlräume des medizinischen Instruments von Flüssigkeitsablagerungen befreit wird.

Falls nach einem Flüssigkeitsaustrag die Reinigungsvorrichtung um näherungsweise 180° in Schwerkraftrichtung von der ersten zur zweiten Betriebsposition geschwenkt wird, ist die Reinigungsvorrichtung noch mit Flüssigkeit gefüllt. Erst nach Abschluss der analog zu den in Figuren 3a bis 3d gezeigten Schritte ist die Reinigungsvorrichtung mit Luft gefüllt. Eine mehrmalige Ausführung der Schritte analog zu Figur 3a bis 3d ist notwendig um die Flüssigkeit aus dem Instrumentenhohlraum zu entfernen.

Obwohl die Erfindung durch die Darstellung spezifischer Ausführungsbeispiele beschrieben worden ist, ist es offensichtlich, dass zahlreiche weitere Ausführungsvarianten in Kenntnis der vorliegenden Erfindung geschaffen werden können, beispielsweise indem die Merkmale der beispielhaften Ausführungsvarianten miteinander kombiniert und/oder einzelne Funktionseinheiten dieser Ausführungsvarianten ausgetauscht werden.

### Bezugszeichenliste

- 1: Zylindergehäuse
- 2: Zylinder
- 3: Kolbengriff
- 4: Hohlkolben
- 5: Adapter bzw. erstes Befestigungsmodul
- 6: Zweites Befestigungsmodul
- 7: Behälter
- 8: Sicherungskerbe für Zylinder (2)
- 9: Positionierungsüberstand
- 10: Sicherungsnut des zweiten Befestigungsmoduls (6)
- 11a: Stützplatte für Einlassventilfeder (12)
- 11b: Stützplatte für Einlassventilfeder (12), gezeigt als Kontur
- 12: Einlassventilfeder
- 13: Zylinderraum
- 14: Einlassöffnung Kolbenraum
- 15: Erste Stützbühne für Hohlkolbenfeder
- 16: Einlassventilraum
- 17: Einlassventilzylinder
- 18: Anatomischer Bogenlauf der Zylindergehäuse
- 19: Einlassöffnung Hohlkolbe (4)
- 20: Zylinderwand
- 21: Dritter Dichtungsring
- 22: Hohlkolbenkopf
- 23: Hohlkolbenfeder
- 24: Sicherungsüberstrand für Zylinder (2)
- 25: Hohlkolbeninnenraum
- 26: Zweite Stützbühne für Hohlkolbenfeder
- 27: Kolbenöffnung
- 28: Auslassventilkugel
- 29: Auslassventilöffnung
- 30: Sicherungsüberstrand für Kolbengriff (3)
- 31: Auslassventilraum
- 32: Auslassventilfeder
- 33: Anatomischer Bogenlauf des Kolbengriffs (3)
- 34: Sicherungskerbe für den Kolbengriff (3)
- 36: Auslassöffung
- 37: Düse
- 38: Erster Dichtring
- 39: Zweiter Dichtring
- 40: Seitenöffnung
- 41: Sicherungsüberstrand für Adapter (5)
- 42: Sicherungskerbe für Düse (37)
- 43: Adapterraum, Raum des ersten Befestigungsmoduls
- 44: Auslassventilfederstützplatte
- 45: Stützbühne für die Kugel des Auslassventil (28)
- 46: Vierter Dichtring
- 47: Positionierungskerbe
- 48: Flaschengewinde
- 49: Gewindeeinsatz
- 50: Auslassöffnung des Lufteinlasssystems
- 51: Einlassöffnung des Lufteinlasssystems
- 52: Luftkanal
- 53: Luftkanalabschnitt
- 54: Raum Lufteinlasssystem
- 55: Einlassventilkugel
- 56: Sicherungsüberstrand für zweites Befestigungsmodul (6)
- 57: Stützbühne für die Kugel des Einlassventils (55)
- 58: Öffnung zum Flüssigkeitsdurchlass zwischen Behälter und Vorrichtung
- 59: Behälterhohlraum
- 60: Medizinisches Instrument
- 61: Arbeitskopf
- 62: Instrumentenhohlraum (Hauptkanal)
- 63: Einlassöffung des Hauptkanals
- 64: Nebenkanal
- 65: Einlassöffung des Nebenkanals
- 66: Auslassöffung des Hauptkanals
- 67: Auslassöffung des Nebenkanals
- 68: Innerraum zwischen ersten und zweitem Dichtring (38, 39)
- 69: Erstes Schlauchende
- 70: Schlauch
- 71: Zweites Schlauchende
- 72: Zwischenelement
- 73: Sicherungsnut
- 74: Düse
- 75: Gewindeeinsatz
- 79: Dosierpumpe
- 80: Flüssigkeit
- 81: Luft
- 100: Reinigungsvorrichtung

## Patentansprüche

1. Vorrichtung (100) zur Reinigung von medizinischen Instrumenten (60) mit mindestens einem Instrumentenhohlraum (62) und mindestens zwei Öffnungen (63, 66), wobei die Vorrichtung (100) ein erstes Befestigungsmodul (5) zur lösbaren Befestigung des medizinischen Instruments (60) und ein zweites Befestigungsmodul (6) zur lösbaren Befestigung eines ersten Behälters (7) zur Aufnahme von Flüssigkeit (80) und/oder Luft (81) aufweist, dabei weist die Vorrichtung (100) einen Reinigungsvorrichtungshohlraum (25, 13, 16) auf, der derart ausgestaltet ist, dass bei befestigtem medizinischem Instrument (60) und befestigtem Behälter (7) die Flüssigkeit (80) und/oder die Luft (81) vom Innern des Behälter (7) durch den Reinigungsvorrichtungshohlraum (25, 13, 16) in den Instrumentenhohlraum (62) spülbar ist, **dadurch gekennzeichnet, dass** die Vorrichtung (100) eine erste und eine zweite Betriebsposition aufweist, wobei in der ersten Betriebsposition die Flüssigkeit (80) aus dem Behälter (7) durch den Reinigungsvorrichtungshohlraum (25, 13, 16) in den Instrumentenhohlraum (62) spülbar ist und in der zweiten Betriebsposition Luft aus der Umgebung durch den Behälter (7) und den Reinigungsvorrichtungshohlraum (25, 13, 16) in den Instrumentenhohlraum (62) spülbar ist.

2. Vorrichtung (100) nach einem der vorangehenden Ansprüche, **dadurch gekennzeichnet, dass** durch eine Verschwenkung der Vorrichtung (100) um näherungsweise 180° in Schwerkraftrichtung zwischen der ersten und zweiten Betriebsposition gewechselt werden kann, wobei ist in der ersten Betriebsposition die Flüssigkeit auf Grund der Schwerkraft durch das zweite Befestigungsmodul (6) in den Reinigungsvorrichtungshohlraum (25, 13, 16) spülbar.

3. Vorrichtung (100) nach einem der vorangehenden Ansprüche, **dadurch gekennzeichnet, dass** die Vorrichtung (100) eine Dosierpumpe (79) aufweist, wobei mittels der Dosierpumpe (79) in der ersten Betriebsposition Flüssigkeit (80) aus dem Behälter (7) durch den Reinigungsvorrichtungshohlraum (25, 13, 16) in den Instrumentenhohlraum (62) spülbar ist und mittels der Dosierpumpe (79) in der zweiten Betriebsposition Luft aus der Umgebung durch den Behälter (7) und den Reinigungsvorrichtungshohlraum (25, 13, 16) in den Instrumentenhohlraum (62) spülbar ist.

4. Vorrichtung nach einem der vorangehenden Ansprüche, **dadurch gekennzeichnet, dass** ein zweites Aufnahmeelement am zweiten Befestigungsmodul befestigbar ist und das zweite Aufnahmeelement ein Auswahlmodul aufweist, das die wahlweise Entnahme von Flüssigkeit und/oder Gas aus dem ersten oder zweiten Aufnahmeelement ermöglicht.

5. Vorrichtung nach dem Ansprüch 4, **dadurch gekennzeichnet, dass** das Auswahlmodul eine drehbare Scheibe mit mindestens einer Öffnung aufweist, wobei die Öffnung derart ausgestaltet ist, dass die Flüssigkeit und/oder das Gas wahlweise aus dem ersten oder dem zweiten Aufnahmeelement entnehmbar ist.

6. Vorrichtung nach einem der vorangehenden Ansprüche, **dadurch gekennzeichnet, dass** das erste Befestigungsmodul eine Mechanismus zur Rotation beweglicher Teile des medizinischen Instruments aufweist.

7. Vorrichtung nach einem der vorangehenden Ansprüche, **dadurch gekennzeichnet, dass** das erste Befestigungsmodul einen Luer Lock Anschluss aufweist.

8. Vorrichtung nach einem der vorangehenden Ansprüche, **dadurch gekennzeichnet, dass** das erste Befestigungsmodul ein Adapter ist.

9. Vorrichtung nach einem der vorangehenden Ansprüche, **dadurch gekennzeichnet, dass das** zweiten Befestigungsmodul (49) Mittel zur Ausgleich des Drucks im Innern des Behälters (7) aufweist.

10. Verfahren zur Reinigung eines medizinischen Instruments (60) unter Verwendung einer Vorrichtung nach einem der Ansprüche 1 bis 9.

11. Verfahren nach Anspruch 10, **dadurch gekennzeichnet, dass** die Flüssigkeit eine grössere Dichte als die atmosphärische Luft aufweist und sich die Flüssigkeit in der ersten Betriebsposition vor dem zweiten Befestigungsmodul (6) befindet und in der zweiten Betriebsposition sich die atmosphärische Luft vor dem zweiten Befestigungsmodul (6) befindet.

12. Verfahren nach einem der Ansprüche 10 bis 11, **dadurch gekennzeichnet, dass** die Flüssigkeit mehrere Komponenten aufweist und diese Komponenten eine inhomogene Mischung bilden.

13. Verfahren nach einem der Ansprüche 10 bis 12, **dadurch gekennzeichnet, dass** eine der Komponenten Öl ist und der Volumenanteil des Öls in der Flüssigkeit grösser als 0.2 % und kleiner als 10 % ist.

14. Verfahren nach einem der Ansprüche 10 bis 13, **dadurch gekennzeichnet, dass** der Volumenanteil des Öls in der Flüssigkeit grösser als 0.3 % und kleiner als 0.7 % ist.

15. Verfahren nach einem der Ansprüche 10 bis 14, **dadurch gekennzeichnet, dass** eine der Komponenten aus kurzkettige Alkohole besteht und der Volumenanteil der kurzkettigen Alkohole in der Flüssigkeit grösser als 40 % und kleiner als 80 % ist.

16. Verfahren nach einem der Ansprüche 10 bis 15, **dadurch gekennzeichnet, dass** die Flüssigkeit Phosphorsäure aufweist und der Volumenanteil der Phosphorsäure in der Flüssigkeit grösser als 0.1 % und kleiner als 2 % ist.

## Claims

1. A device (100) for cleaning medical instruments (60) having at least one instrument cavity (62) and at least two openings (63, 66), the device (100) comprising a first attachment module (5) for releasably attaching the medical instrument (60) and a second attachment module (6) for releasably attaching a first tank (7) for receiving fluid (80) and/or air (81), the device (100) comprising a cleaning device cavity (25, 13, 16) designed such that the fluid (80) and/or the air (81) can be flushed from the interior of the tank (7), through the cleaning device cavity (25, 13, 16) into the instrument cavity (62) when the medical instrument (60) is attached and the tank (7) is attached, **characterized in that** the device (100) comprises a first and a second operating position, wherein in the first operating position the fluid (80) can be flushed from the tank (7), through the cleaning device cavity (25, 13, 16) and into the instrument cavity (62) and in the second operating position air from the environment can be flushed through the tank (7), through the cleaning device cavity (25, 13, 16) and into the instrument cavity (62).

2. The device (100) according to one of the preceding claims, **characterized in that** pivoting the device (100) by approximately 180° in the direction of gravity causes a switch from the first to the second operating position, wherein in the first position the fluid can be flushed through the second attachment module (6) into the cleaning device cavity (25, 13, 16) owing to gravity.

3. The device (100) according to any one of the preceding claims, **characterized in that** the device (100) comprises a metering pump (79), wherein in the first operating position fluid (80) can be flushed out of the tank (7), through the cleaning device cavity (25, 13, 16) and into the instrument cavity (62) by means of the metering pump (79) and in the second operating position air can be flushed from the environment, through the tank (7), through the cleaning device cavity (25, 13, 16) and into the instrument cavity (62) by means of the metering pump (79).

4. The device according to any one of the preceding claims, **characterized in that** a second receiving element can be attached to the second attachment module, and the second receiving element comprises a selector module allowing fluid and/or gas to be withdrawn selectively from the first or the second receiving element.

5. The device according to claim 4, **characterized in that** the selector module comprises a rotating disc having at least one opening, wherein the opening is designed such that the fluid and/or the gas can be withdrawn selectively from the first or the second receiving element.

6. The device according to any one of the preceding claims, **characterized in that** the first attachment module comprises a mechanism for rotating displaceable parts of the medical instrument.

7. The device according to any one of the preceding claims, **characterized in that** the first attachment module comprises a Luer Lock connector.

8. The device according to any one of the preceding claims, **characterized in that** the first attachment module is an adapter.

9. The device according to any one of the preceding claims, **characterized in that** the second attachment module (49) comprises means for equalizing the pressure in the interior of the tank (7).

10. A method for cleaning a medical instrument (60) using a device according to any one of the claims 1 through 9.

11. The method according to claim 10, **characterized in that** the fluid comprises a greater density than atmospheric air and that the fluid is located ahead of the second attachment module (6) in the first operating position and the atmospheric air is located before the second attachment module (6) in the second operating position.

12. The method according to either of the claims 10 through 11, **characterized in that** the fluid comprises a plurality of components and said components form a non-homogenous mixture.

13. The method according to any one of the claims 10 through 12, **characterized in that** one of the components is oil and the volume content of the oil in the fluid is greater than 0.2% and less than 10%.

14. The method according to any one of the claims 10 through 13, **characterized in that** the volume content of the oil in the fluid is greater than 0.3% and less than 0.7%.

15. The method according to any one of the claims 10 through 14, **characterized in that** one of the components is made from short-chain alcohols and the volume content of the short-chain alcohols in the fluid is greater than 40% and less than 80%.

16. The method according to any one of the claims 10 through 15, **characterized in that** the fluid comprises phosphoric acid and the volume content of the phosphoric acid in the fluid is greater than 0.1% and less than 2%.

## Revendications

1. Dispositif (100) pour nettoyer des instruments médicaux (60), avec au moins un compartiment à instrument creux (62) et au moins deux ouvertures (63, 66), sachant que le dispositif (100) comporte un premier module de fixation (5) pour la fixation amovible de l'instrument médical (60) et un second module de fixation (6) pour la fixation amovible d'un premier récipient (7) pour la réception de liquide (80) et/ou d'air (81), le dispositif (100) présentant un compartiment creux de dispositif de nettoyage (25, 13, 16) qui se présente sous une forme telle que lorsque l'instrument médical (60) et le récipient (7) sont fixés, le liquide (80) et/ou l'air (81) puisse(nt) être refoulé(s) de l'intérieur du récipient (7) à travers le compartiment creux de dispositif de nettoyage (25, 13, 16) dans le compartiment creux à instruments (62), **caractérisé en ce que** le dispositif (100) présente une première et une seconde positions de fonctionnement, sachant que dans la première position de fonctionnement, le liquide (80) peut être refoulé depuis le récipient (7) à travers le compartiment creux de dispositif de nettoyage (25, 13, 16) dans le compartiment creux à instruments (62) et dans la seconde position de fonctionnement, l'air peut être refoulé des alentours à travers le récipient (7) et le compartiment creux de dispositif de nettoyage (25, 13, 16) et dans le compartiment creux à instruments (62).

2. Dispositif (100) selon la revendication précédente, **caractérisé en ce qu'**il est possible de commuter entre la première et la seconde positions de fonctionnement par pivotement du dispositif (100) de 180° approximativement dans le sens de la gravité, sachant qu'à la première position de fonctionnement, le liquide peut être refoulé à travers le second module de fixation (6) dans le compartiment creux de dispositif de nettoyage (25, 13, 16) sous l'effet de la gravité.

3. Dispositif (100) selon l'une des revendications précédentes, **caractérisé en ce que** le dispositif (100) comporte une pompe doseuse (79), sachant qu'à l'aide de la pompe doseuse (79), à la première position de fonctionnement, le liquide (80) peut être refoulé depuis le récipient (7) à travers le compartiment creux de dispositif de nettoyage (25, 13,16) dans le compartiment creux à instruments (62) et à l'aide de la pompe doseuse (79), dans la seconde position de fonctionnement, l'air peut refoulé des alentours, à travers le récipient (7) et le compartiment creux de dispositif de nettoyage (25, 13, 16) dans le compartiments à instruments (62).

4. Dispositif selon l'une des revendications précédentes, **caractérisé en ce qu'**un second élément de réception peut être fixé au second module de fixation et le second élément de réception comporte un module de sélection, qui permet le prélèvement, au choix, de liquide et/ou de gaz depuis le premier ou le second élément de réception.

5. Dispositif selon la revendication 4, **caractérisé en ce que** le module de sélection comporte un disque rotatif avec au moins une ouverture, sachant que l'ouverture se présente sous une forme telle que le liquide et/ou le gaz puisse(nt) être prélevé(s) au choix depuis le premier ou le second élément de réception.

6. Dispositif selon l'une des revendications précédentes, **caractérisé en ce que** le premier module de fixation comporte un mécanisme pour la rotation de pièces mobiles de l'instrument médical.

7. Dispositif selon l'une des revendications précédentes, **caractérisé en ce que** le premier module de fixation comporte un raccord Luer Lock.

8. Dispositif selon l'une des revendications précédentes, **caractérisé en ce que** le premier module de fixation est un adaptateur.

9. Dispositif selon l'une des revendications précédentes, **caractérisé en ce que** le second module de fixation (49) comporte des moyens pour la compensation de la pression à l'intérieur du récipient (7).

10. Procédé pour nettoyer un instrument médical (60) en utilisant un dispositif selon l'une des revendications 1 à 9.

11. Procédé selon la revendication 10, **caractérisé en ce que** le liquide présente une densité supérieure à celle de l'air atmosphérique et, à la première position de fonctionnement, le liquide se trouve en amont du second module de fixation (6) et, à la deuxième position de fonctionnement, l'air atmosphérique se trouve en amont du second module de fixation (6).

12. Procédé selon l'une des revendications 10 à 11, **caractérisé en ce que** le liquide présente plusieurs composants et ces composants constituent un mélange hétérogène.

13. Procédé selon l'une des revendications 10 à 12, **caractérisé en ce que** l'un des composants est de l'huile et le pourcentage volumique de l'huile dans le liquide est supérieur à 0,2 % et inférieur à 10 %.

14. Procédé selon l'une des revendications 10 à 13, **caractérisé en ce que** le pourcentage volumique de l'huile dans le liquide est supérieur à 0,3 % et inférieur à 0,7 %.

15. Procédé selon l'une des revendications 10 à 14, **caractérisé en ce que** l'un des composants se compose d'alcools à chaîne courte et le pourcentage volumique des alcools à chaîne courte dans le liquide est supérieur à 40 % et inférieur à 80 %.

16. Procédé selon l'une des revendications 10 à 15, **caractérisé en ce que** le liquide contient de l'acide phosphorique et le pourcentage volumique de l'acide phosphorique dans le liquide est supérieur à 0,1 % et inférieur à 2 %.
